# EUROPÄISCHE PATENTSCHRIFT

(11) **EP 4 039 791 B1**
(45) Veröffentlichungstag und Bekanntmachung des Hinweises auf die Patenterteilung: **30.08.2023**
(21) Anmeldenummer: 21217070.8
(22) Anmeldetag: 22.12.2021
(51) Int. Cl.: G06N 3/08, C12M 1/36

(54) **APPARATUR ZUM STEUERN EINES PROZESSES SOWIE ZUGEHÖRIGES STEUERUNGSVERFAHREN**
DEVICE FOR CONTROLLING A PROCESS AND CORRESPONDING CONTROL METHOD
APPAREIL DE COMMANDE D'UN PROCESSUS, AINSI QUE PROCÉDÉ DE COMMANDE ASSOCIÉ

(30) Priorität: 13.01.2021 DE 102021100531
(43) Veröffentlichungstag der Anmeldung: 10.08.2022
(73) Patentinhaber: Biothera Institut GmbH, 79102 Freiburg (DE)
(72) Erfinder: Mertelsmann, Roland, 79104 Freiburg (DE); Bödecker, Joschka, 79108 Freiburg (DE); Raith, Dennis, 70195 Stuttgart (DE); Bermeitinger, Jonas, 79418 Schliengen (DE)
(74) Vertreter: Mertzlufft-Paufler, Cornelius

(56) Entgegenhaltungen:
- WO-A1-2009/039433
- WO-A1-2020/016223
- WO-A1-2020/086635
- US-A1- 2017 138 924
- US-B2- 10 042 334
- MANTRIPRAGADA VENKATA P ET AL: "Automated in-process characterization and selection of cell-clones for quality and efficient cell manufacturing", CYTOTECHNOLOGY, SPRINGER NETHERLANDS, DORDRECHT, Bd. 72, Nr. 5, 30. Juni 2020 (2020-06-30), Seiten 615-627, XP037264961, ISSN: 0920-9069, DOI: 10.1007/S10616-020-00403-W [gefunden am 2020-06-04]

## Beschreibung

Die Erfindung betrifft eine Apparatur zum autonomen Steuern eines Prozesses. Ein solcher Prozess kann insbesondere ein biologischer, oder aber auch ein chemischer oder physikalischer Prozess sein. Der Prozess kann in bestimmten Anwendungen auch ein Umwandlungsprozess sein, bei dem aus Ausgangsstoffen oder Ausgangsverbindungen neue Produkte, zum Beispiel in Form neuer Stoffe oder Verbindungen oder in Form neuer physikalischer Objekte, etwa Partikel bestimmter Form und/oder Größe, gewonnen werden.

Die Erfindung betrifft ferner ein zugehöriges Verfahren zum Steuern einer Apparatur, in der ein solcher Prozess abläuft, wobei der Prozess durch Einstellen mindestens eines Wirkparameters gesteuert wird.

Der besagte Prozess kann insbesondere von einem biologischen System ausgeführt werden. Das biologische System kann beispielsweise von Zellen und/oder Mikroorganismen und/oder Enzymen gebildet sein. Das biologische System kann daher insbesondere eine Vielzahl von Elementen aufweisen. Die Elemente können beispielsweise Zellen und/oder Mikroorganismen und/oder Enzyme sein. Das biologische System kann in einem Kulturmedium eingebettet sein und zusammen mit diesem eine biologische Probe bilden. Beispielsweise kann das Kulturmedium ein Nährstoffbett für ein aus Zellen bestehendes biologisches System sein.

Der biologische Prozess kann insbesondere ein Wachstum oder ein Stoffwechsel oder eine biologische Funktion einer Zelle sein, wobei die Zelle zum Beispiel in einem Kulturmedium innerhalb eines Kulturraums der Apparatur kultiviert werden kann. Bei der Zelle kann es sich beispielsweise um eine Krebszelle oder um einen Mikroorganismus handeln. Bei dem biologischen Prozess kann es sich insbesondere um einen mikrobiologischen Prozess handeln.

Alternativ hierzu kann der Prozess auch ein chemischer oder ein rein physikalischer Prozess sein, etwa zur Gewinnung bestimmter mikroskopischer Partikel.

Eingangs beschriebene Verfahren und Vorrichtungen sind in Grundzügen bereits vorbekannt. Beispielsweise sind Verfahren bekannt, mit welchen die Zusammensetzung einer Nährlösung oder anderer Faktoren, die das Wachstum von Zellen einer biologischen Probe beeinflussen, von einer Apparatur zur Kultivierung der Zellen eingestellt wird, wobei die Apparatur selbständig erlernt hat, wie diese Faktoren einzustellen sind, um ein optimales Wachstum zu ermöglichen. Die konkrete Auswahl der zu verändernden Prozessfaktoren, etwa einer Heizleistung zum Einstellen einer globalen Temperatur der biologischen Probe, und deren Überwachung / Regelung mittels der Apparatur hat sich jedoch in der Praxis als herausfordernd und nicht immer reproduzierbar erwiesen.

WO 2020 016223 A1 beschreibt eine Apparatur für biologische Prozesse sowie ein Verfahren zur Einrichtung einer solchen Apparatur. Um dazu computergestützt Prozessparameter vorgeben und damit den biologischen Prozess steuern zu können, wird vorgeschlagen, einen Prozesszustand automatisch zu erfassen und diesen erfassten Prozesszustand anhand einer vorgegebenen Zielvorgabe computergestützt zu bewerten. Unter einem Prozesszustand versteht die WO 2020 016223 A1 dabei den momentanen Zustand, in dem sich das biologisches System, welches den Prozess ausführt, gerade befindet. Dieser Prozesszustand kann beispielsweise über die Messung einer Zelldichte oder einer Anzahl an lebendigen Zellen erfasst werden.

Aus WO 2020 086635 A1 ist ferner eine Methode vorbekannt, bei der mittels Raman-Spektroskopie spektrale Messdaten eines biologischen Systems, welches sich in einem Bioreaktor befindet, erfasst werden. Auf Basis dieser gemessenen spektralen Daten werden dann computergestützt aus einer Datenbank passende relevante zugehörige analytische Messdaten abgerufen, die in der Datenbank hinterlegt sind. Auf Basis der von der Datenbank abgerufenen analytischen Messdaten wird dann ein lokales Modell erstellt, mit dem sich analytische Messwerte des biologischen Systems anhand der erfassten spektralen Messdaten abschätzen lassen, sodass diese analytischen Messdaten nicht länger real und aufwändig gemessen werden müssen. Dieser Ansatz erlaubt es, anhand der geschätzten bzw. vorhergesagten analytischen Messdaten eine closed-loop Regelung des im Bioreaktor ablaufenden biologischen Prozesses zu implementieren, wobei diese Regelung die geschätzten analytischen Messdaten als Eingangsgrößen verwendet und die Regelung selbst nicht adaptiv ausgestaltet ist.

Ausgehend von diesem vorbekannten Stand der Technik liegt der Erfindung die Aufgabe zu Grunde, eine verbesserte, das heißt insbesondere stabilere und präzisere, Steuerung von Prozessen mit wie eingangs erwähnten Apparaturen zu ermöglichen.

Zur Lösung dieser Aufgabe sind erfindungsgemäß bei einem Verfahren zum Steuern einer Apparatur, in der ein wie eingangs erläuterter Prozess abläuft, die Merkmale von Anspruch 1 vorgesehen. Es wird somit erfindungsgemäß zur Lösung der Aufgabe bei einem Verfahren der eingangs genannten Art vorgeschlagen, dass eine Prozessantwort des Prozesses, die der Prozess in Reaktion auf den mindestens einen Wirkparameter zurückgibt, gemessen wird, dass diese Prozessantwort mittels einer Bewertung computerimplementiert anhand einer vorgegebenen Zielvorgabe bewertet wird und dass mindestens ein Einstellwert des mindestens einen Wirkparameters computerimplementiert anhand der Bewertung angepasst wird, wobei mindestens ein optimierter Einstellwert für den mindestens einen Wirkparameter computerimplementiert von der Apparatur selbständig anhand mehrerer Bewertungen erlernt wird, die die Apparatur aus einer jeweiligen Prozessantwort, jeweils in Reaktion auf von der Apparatur vorgegebene Einstellwerte des mindestens einen Wirkparameters, abgeleitet hat.

Die Prozessantwort kann hier somit als eine Antwort des (biologischen / chemischen / physikalischen) Systems verstanden werden, welches für den besagten Prozess verantwortlich ist / diesen ausführt. Durch die Messung der Prozessantwort wird dabei ein Verbrauch und/oder eine Produktion mindestens eines Stoffs durch den Prozess, zumindest indirekt, erfasst und/oder es wird hierdurch eine Aktivität eines biologischen Systems, welches den Prozess ausführt, erfasst.

Mit anderen Worten wird gemäß der Erfindung somit zur Lösung der eingangs genannten Aufgabe bei einem Verfahren der eingangs genannten Art vorgeschlagen, dass erfasst wird, wie der Prozess auf eine Anpassung von wenigstens einem Wirkparameter (mit dem der Prozess steuerbar ist) reagiert. Hierbei wird die Reaktion des Prozesses (also beispielsweise insbesondere eines biologischen Systems, welches durch die beteiligten Zellen und/oder Mikro-Organismen gebildet ist) als eine Prozess- oder Systemantwort aufgezeichnet und mit einer Bewertung bewertet, um anhand der Bewertung eine erneute Anpassung des Wirkparameters vorzunehmen. Dadurch kann eine Regelschleife aufgebaut werden, die den Prozess kontinuierlich steuert und zwar autonom, ohne jegliches Eingreifen von außen. Beispielsweise lässt sich dieses Verfahren mit einer erfindungsgemäßen Apparatur gemäß Anspruch 17 umsetzen die unten noch genauer beschrieben wird.

Der Wirkparameter kann insbesondere ein Prozessparameter sein, mit dem Einfluss auf den Prozess genommen werden kann. Hierzu zählen beispielsweise Umweltbedingungen wie eine Temperatur, ein Druck oder eine Feuchtigkeit. Ein Wirkparameter kann beispielsweise auch durch einen Volumenstrom eines Stoffes gegeben sein, welcher dem Prozess zugeführt wird.

Die Einstellung von Wirkparametern kann dabei auch umfassen, die Wirkparameter zu mehreren Zeitpunkten oder zeitlich kontinuierlich und veränderbar einzustellen.

Es kann vorgesehen sein, dass eine Mehrzahl oder eine Vielzahl von (insbesondere biologischen) Prozessen parallel und/oder seriell in der Apparatur ablaufen. Es kann von Vorteil sein, wenn für mindestens zwei parallel ablaufende Prozesse unterschiedliche Wirkparameter eingestellt werden. Die Wirkparameter können in alternativen Experimenten auch gleich eingestellt werden.

Weiter kann vorgesehen sein, dass während des Ablaufs eines Prozesses mehrfach dessen Prozessantwort und bevorzugt zusätzlich dessen Prozesszustand erfasst und/oder bewertet wird. Dies erfolgt bevorzugt kontinuierlich.

Das obige Verfahren kann in verschiedenen Anwendungsfällen zum Einsatz kommen: In einem ersten Fall kann das Verfahren genutzt werden, um eine wie eingangs beschriebene Apparatur initial einzurichten, also um Einrichtungsparameter für den mindestens einen Wirkparameter zu ermitteln. Ist die Apparatur eingerichtet, so ist die Apparatur in sehr effizienter Weise dazu in der Lage, einen (insbesondere biologischen) Prozess anhand der ermittelten Einrichtungsparameter präzise zu steuern, selbst wenn der Prozess sich leicht von dem Prozess unterscheidet, der für die Einrichtung der Apparatur genutzt wurde. Im ersten Fall wird das Verfahren somit zum initialen Einrichten der Apparatur genutzt.

Im zweiten Fall hingegen wird das Verfahren zur fortlaufenden Steuerung der Apparatur genutzt. In dem zweiten Fall kann die Apparatur hocheffizient genutzt werden und ermöglicht insbesondere durch die Steuerung einer Vielzahl von parallel ablaufenden, gleichartigen Prozessen, einen hohen Informationsgewinn über den jeweiligen Prozess in kürzester Zeit, was insbesondere für die Anwendung auf biologische Prozesse von hohem Interesse ist. Hierdurch kann beispielsweise die Entwicklung von Medikamenten in hohem Maß beschleunigt werden.

Von Vorteil ist in beiden Fällen, dass ohne oder mit minimalem personellen Aufwand ein jeweiliger Prozess, vorzugsweise autonom, mittels der Apparatur und unter Anwendung des erfindungsgemäßen Verfahrens zu einem gewünschten Verhalten geführt werden kann. Das gewünschte Verhalten kann in Bezug auf unterschiedlichste Parameter bestehen, etwa eine Überlebensfähigkeit oder Unterdrückung von Zellen oder ein Wachstum / eine Vermehrung von Zellen (im Falle eines biologischen Prozesses), eine gewünschte Stoffproduktion oder eine bestimmte Stoffumsetzung (im Falle eines chemischen Prozesses oder (z.B. durch Verstoffwechselung eines abzubauenden Stoffs in Zellen oder die Produktion eines bestimmten Stoffes durch die Zellen) oder etwa die Formierung von Emulsionströpfchen mit bestimmten Eigenschaften (im Falle eines physikalischen Prozesses).

Durch die Anpassung des Wirkparameters können Bedingungen, insbesondere Umgebungsbedingungen oder Wachstums- oder Ernährungsbedingungen, verändert beziehungsweise geschaffen werden, die den (insbesondere biologischen) Prozess beeinflussen und somit steuern. Dadurch kann der Prozess in Richtung eines gewünschten Systemzustands gelenkt werden. Im Falle eines biologischen Prozesses, der insbesondere von einem von Zellen gebildeten biologischen System ausgeführt wird, kann etwa eine bestimme Reaktion auf diese Bedingungen als Prozess- oder Systemantwort ausgegeben werden. Dies bedeutet, dass die Prozessantwort erfasst wird, um daraus Rückschlüsse über einen momentanen Zustand des biologischen Prozesses oder des jeweiligen dem Prozess zugrundeliegenden Systems zu gewinnen.

Die vorgenommene Anpassung kann dabei zu einer Optimierung des mindestens einen Wirkparameters führen hinsichtlich einer verbesserten Prozessantwort. Beispielsweise kann die Prozessantwort nach Anpassung / Optimierung des mindestens einen Wirkparameters näher an eine Zielvorgabe heranrücken. Eine solche Zielvorgabe kann, zum Beispiel über Vor-Experimente, etwa von einem Prozesszustand eines biologischen Prozesses, abgeleitet sein. Hierbei kann der jeweilige Prozess gerade mit dem Verfahren erreicht beziehungsweise reproduzierbar eingestellt werden.

Die Zielvorgabe kann ein Bewertungskriterium zur Bewertung der jeweiligen Prozessantwort und/oder des jeweiligen Prozesszustands bilden oder es kann vorgesehen sein, dass aus der Zielvorgabe ein solches Bewertungskriterium abgeleitet wird. Bei dem Bewertungskriterium kann es sich beispielsweise um eine Belohnungs-, Kosten-, oder Bestrafungsfunktion handeln.

So kann beispielsweise aus der genannten Zielvorgabe ein Bewertungskriterium abgeleitet werden, welches vorsieht, dass eine Abweichung der gemessenen Prozessantwort von einer Referenzantwort bewertet wird.

Soll hingegen der momentane Zustand des (insbesondere biologischen) Prozesses bewertet werden, kann beispielsweise eine Zelldichte, etwa von lebenden Krebszellen, als ein Zustandskriterium verwendet werden.

Es kann auch vorgesehen sein, dass die Zielvorgabe einen Einstellparameter bewertet, wie etwa die in einem bestimmten Zeitraum applizierte Menge eines Medikaments.

Die Zielvorgabe kann beispielsweise eine gewichtete Belohnungsfunktion vorgeben. Es kann insbesondere vorgesehen sein, dass die Zielvorgabe einen Zustand in den Kategorien besser oder schlechter bewertet. Es kann vorgesehen sein, dass eine Belohnungsfunktion vorgegeben ist, die reelle Zahlen in einem Intervall annehmen kann, beispielsweise zwischen 0 und 1, wobei eine erste reelle Zahl, die näher bei 1 liegt als eine zweite reelle Zahl, besser bewertet wird als die zweite reelle Zahl.

Es kann auch vorgesehen sein, dass die Zielvorgabe eine Belohnungsfunktion vorsieht, welche lediglich zwei Zustände, beispielsweise 1 und 0 oder unendlich und 0 oder dergleichen aufweist. Es kann insbesondere vorgesehen sein, dass die Zielvorgabe einen Zustand in den Kategorien gut oder schlecht bewertet. Es kann vorgesehen sein, dass eine Belohnungsfunktion maximiert werden soll.

Äquivalent hierzu kann vorgesehen sein, dass die Belohnungsfunktion eine Kostenfunktion ist, welche minimiert werden soll. Beispielsweise kann hierbei eine 0 keine Kosten verursachen und eine 1 hohe Kosten. Eine Kostenfunktion kann beispielsweise eine Anzahl von Zeitschritten abbilden. Dies kann beispielsweise zweckmäßig sein, wenn es ein Ziel ist, eine zeitoptimale Lösung zu finden, beispielsweise weil eine Referenzantwort zeitlich veränderlich vorgegeben ist und eine solche möglichst von der Prozessantwort erreicht werden soll.

Das Erfassen der Prozessantwort kann bevorzugt automatisiert, insbesondere computerimplementiert erfolgen oder aber manuell, etwa in regelmäßigen Abständen.

Das Erfassen der Prozessantwort, ihre Bewertung, das Einstellen des mindestens einen Wirkparameters sowie alle übrigen zuvor erläuterten Maßnahmen können computerimplementiert erfolgen, bevorzugt mittels einer künstlichen Intelligenz (KI), die noch genauer beschrieben wird. Diese KI kann auch die Zielvorgabe, insbesondere einzelne Zielgrößen der Zielvorgabe, definieren.

Der mindestens eine Wirkparameter kann beispielsweise eine ganze Reihe von unterschiedlichen Wirkparametern umfassen. Diese können zum Beispiel in einem Wirkungsvektor zusammengefasst werden, dessen einzelne Komponenten durch die jeweiligen Wirkparameter gebildet sind. Jeder Wirkparameter kann dabei zum Einstellen einer jeweiligen Regelgröße der Apparatur eingesetzt werden. Somit kann der Wirkungsvektor einen bestimmten momentanen Regelzustand beschreiben, der in der Apparatur eingestellt ist, um den jeweiligen (insbesondere biologischen) Prozess zu steuern.

Erfindungsgemäß kann die Aufgabe auch durch weitere vorteilhafte Ausführungen gemäß den Unteransprüchen gelöst werden.

Beispielsweise kann die Bewertung der Prozessantwort anhand einer Referenzantwort für die Prozessantwort erfolgen. Diese Referenzantwort kann dann die zuvor genannte Zielvorgabe bilden. Dadurch kann insbesondere erreicht werden, dass die Prozessantwort an die Zielvorgabe angeglichen wird.

Die Zielvorgabe kann entweder deterministisch, etwa aus einem Modell des Prozesses, insbesondere mittels einer Simulation, ermittelt werden. Alternativ kann die Zielvorgabe auch empirisch ermittelt werden, etwa in dem die Prozessantwort des Prozesses in einem Vorexperiment als Zielvorgabe aufgezeichnet wird, wenn sich der Prozess gerade in einem gewünschten Prozesszustand befindet (etwa optimale Stoffwechselproduktion oder ein gewünschter Zelltod von Krebszellen oder ein zu diesem Zeitpunkt gemessenes Spektrum).

Die Referenzantwort kann ferner mehrere Zielgrößen umfassen. Insbesondere kann die Referenzantwort somit eine gemessene und/oder eine virtuelle, insbesondere simulierte oder errechnete Zielgröße umfassen. Virtuelle Zielgrößen können insbesondere von der erwähnten KI bereit gestellt werden.

Bei einer weiteren Ausgestaltung kann vorgesehen sein, dass die Prozessantwort des (insbesondere biologischen) Prozesses auf ein Medium gemessen wird, das Elemente des dem Prozess zugrundeliegenden Systems umgibt, insbesondere wobei das (zum Beispiel biologische) System in das ein Kulturmedium bildende Medium eingebettet ist. So wird durch die Prozessantwort häufig das umgebende Medium verändert. Messdaten zu dem Medium zu erfassen kann daher zu einem wichtigen Informationsgewinn zur Verbesserung der Steuerung beitragen.

Es kann ferner vorgesehen sein, dass der Prozess in einer, insbesondere mikrofluidischen und/oder biologischen Probe abläuft. Diese Probe kann auch ein Kulturmedium umfassen. In diesem Fall kann die Prozessantwort in dem Kulturmedium gemessen werden. Dies bietet sich insbesondere zur Untersuchung von Wachstum oder Stoffwechsel von Mikro-Organismen an.

In wiederum anderen Anwendungen, etwa bei der Steuerung eines biologischen Prozesses zur Produktion von künstlichem Fleisch, kann die biologische Probe eine makroskopische Probe sein, die etwa ein Volumen von mehreren Litern oder gar Hektolitern umfasst. Dies bedeutet, dass das hier vorgestellte Verfahren insbesondere für Anwendungen der Prozessindustrie, insbesondere im Biotech-Bereich, geeignet ist.

Sowohl bei mikroskopischen wie makroskopischen biologischen/chemischen/physikalischen Proben ist es möglich, dass die Messung der Prozessantwort unter Ausschluss des Systems erfolgt. Beispielsweise kann bei einem biologischen System die Messung unter Ausschluss der Zellen und/oder von Mikro-Organismen erfolgen, die in der biologischen Probe enthalten sind, in welcher der Prozess abläuft. Es kann also insbesondere vorgesehen sein, dass mindestens ein Messparameter der Prozessantwort in einer direkten Umgebung der Elemente des (insbesondere biologischen) Systems, insbesondere in einer direkten Umgebung von Zellen und/oder Mikroorganismen, gemessen wird, jedoch gerade ohne direkte Messung der Elemente des Systems.

Wie eingangs erwähnt, kann durch die Messung der Prozessantwort ein Verbrauch und/oder die Produktion mindestens eines Stoffs durch den (insbesondere biologischen) Prozess, zumindest indirekt oder direkt, erfasst werden. Dies kann vorzugsweise geschehen, während auf den Prozess durch Einstellen des Wirkparameters Einfluss genommen wird.

Wie ebenfalls eingangs erwähnt, kann durch Messung der Prozessantwort ferner auch eine Aktivität des Systems, insbesondere eine Aktivität von Zellen und/oder Mikroorganismen im Falle eines biologischen Systems, erfasst werden. Eine solche Aktivität kann zum Beispiel eine Thermogenese (Produktion von Wärme durch Stoffwechselaktivität des biologischen Systems) und/oder eine Chemo- oder Biogenese (Produktion chemischer Substanzen oder biologischer Organismen durch Stoffwechselaktivität) und/oder eine Photogenese (Produktion von Licht durch Stoffwechselaktivität) und/oder ein Energieverbrauch und/oder ein Stoffverbrauch sein, der jeweils auf den Prozess zurückzuführen ist.

Der Prozess, insbesondere das dem Prozess zugrunde liegende System, kann zudem mindestens einen Umgebungsfaktor verändern, wie zum Beispiel eine stoffliche Zusammensetzung, zum Beispiel von Mikroorganismen oder eines sie umgebenden Kulturmediums, und/oder eine Temperatur und/oder ein pH-Wert und/oder eine Permittivität und/oder eine elektrische Leitfähigkeit und/oder ein optisches Transmissions-, Absorptions- oder Reflektionsverhalten, um nur einige Beispiele aufzuzählen.

Diese Umgebungsfaktoren können insbesondere Faktoren eines Kulturmediums sein, in welchem ein biologisches System kultiviert wird. Daher kann vorgesehen sein, dass mindestens ein solcher Umgebungsfaktor als ein Messparameter der Prozessantwort gemessen wird. Dadurch wird erreicht, dass eine Änderung des Umgebungsfaktors in Reaktion auf die Anpassung des mindestens einen Wirkparameters erfasst wird, was wiederum Rückschlüsse auf den momentanen Zustand des (insbesondere biologischen) Prozesses erlaubt.

Gemäß einer besonders vorteilhaften Ausgestaltung ist vorgesehen, dass wenigstens ein Messparameter der Prozessantwort durch eine spektrometrische Messung erfasst wird. Dies ist sowohl auf biologische, chemische und physikalische Prozesse anwendbar.

Im Falle eines biologischen Prozesses kann hierzu vorzugsweise ein Kulturmedium, in welchem das biologische System kultiviert wird, spektrometrisch vermessen werden. Um stets gleichbleibend die Prozessantwort besonders reproduzierbar erfassen zu können, kann vorgesehen sein, dass nur das Kulturmedium, nicht jedoch die darin befindlichen Zellen und/oder Mikroorgansimen durchleuchtet werden. In anderen Situation kann es hingegen vorteilhaft sein, hierzu sowohl das Kulturmedium als auch die darin befindlichen Elemente zu durchleuchten. Dies kann beispielsweise durch eine OD-600-Messung erfolgen, bei welcher eine optische Dichte (OD = optical density), das heißt eine Absorption des Kulturmediums samt darin befindlicher Zellen, bei einer Wellenlänge von 600 nm bestimmt wird.

Die spektrometrische Messung kann unter Verwendung von Beleuchtungslicht im UV und/oder VIS und/oder IR Wellenlängenbereich erfolgen. Ferner kann ein Spektrum, insbesondere ein Transmissions- beziehungsweise Absorptionsspektrum, über einen bestimmten Wellenlängenbereich als ein Messparameter / als Prozessantwort erfasst werden. Als Zielvorgabe kann in diesem Fall ein Zielspektrum verwendet werden. Beispielsweise kann das Zielspektrum in einer Flüssigkeit bestimmt werden, deren Zusammensetzung durch den jeweiligen (chemischen / biologischen / physikalischen) Prozess verändert wird.

Eine bevorzugte Ausgestaltung, die sich insbesondere bei Verwendung einer mikrofluidischen Apparatur anbietet, sieht vor, dass die spektrometrische Messung mittels einer Faseroptik durchgeführt wird. Diese Faseroptik kann in eine mikrofluidische Apparatur integriert sein, in welcher der Prozess abläuft. Dies bietet den Vorteil, dass ein Sensor zur Aufnahme des Spektrums außerhalb der Mikrofluidik angeordnet werden kann, was eine robuste Messlösung ergibt.

Wie bereits erwähnt wurde, können der Prozess und/oder das ihm zugrundeliegende System eine Zusammensetzung eines in der Apparatur verwendeten Kulturmediums verändern. Daher kann auch die Prozessantwort von der Zusammensetzung des Kulturmediums abhängen. Das heißt aber, dass die Prozessantwort mittels einer Untersuchung des Kulturmediums, bevorzugt unter Ausschluss des (insbesondere biologischen) Systems, gemessen werden kann. Die Veränderung der Zusammensetzung des Kulturmediums kann beispielsweise daraus resultieren, dass das System Stoffe des Kulturmediums verbraucht, oder Stoffwechselprodukte in das Kulturmedium abgibt. Dies gilt sowohl für chemische wie biologische Prozesse.

Die Prozessantwort, insbesondere gemessene Messparameter, können dabei so gewählt werden, dass aus der gemessenen Prozessantwort quantitative Rückschlüsse möglich sind auf einen Stoffverbrauch und/oder eine Stoffproduktion durch den Prozess/das System.

Die Prozessantwort kann insbesondere elektrisch und/oder optisch und/oder induktiv erfasst werden. Es kann ferner vorgesehen sein, dass die Erfassung der Prozessantwort eine Entnahme einer (insbesondere biologischen / chemischen / physikalischen) Probe umfasst, wobei die Probe untersucht und daraus die Prozessantwort abgeleitet wird.

Die Erfassung der Prozessantwort kann zudem kontinuierlich oder diskontinuierlich erfolgen.

Schließlich kann anhand der erfassten Prozessantwort die Messung der Prozessantwort selbst zeitlich gesteuert werden. Beispielsweise kann bei einer signifikanten Veränderung der Prozessantwort, oder bei Annähern an Punkte der Prozessantwort, die für die Steuerung des Prozesses kritisch sind, eine zeitliche Frequenz der Erfassung der Prozessantwort angepasst werden, etwa um eine höhere zeitliche Auflösung der Prozessantwort zu erzielen.

Gemäß eines weiteren Aspekts der Erfindung kann mindestens die Anpassung des jeweiligen Einstellwerts des mindestens einen Wirkparameters, vorzugsweise und die vorangegangene Bewertung der gemessenen Prozessantwort, mittels einer künstlichen Intelligenz (KI) computerimplementiert erfolgen. Eine solche KI kann beispielsweise mittels einer Methode des maschinellen Lernens, vorzugsweise mittels einer Methode des mehrschichtigen Lernens (deep learning) und/oder unter Einsatz von künstlichen Neuronalen Netzen (KNN) realisiert sein. Hierbei können die KNNs zahlreiche nicht zugängliche Zwischenschichten (hidden layers) aufweisen.

Die künstliche Intelligenz kann insbesondere modellfrei ausgestaltet sein. Modellfrei bedeutet, dass kein Modell des Prozesses oder der Steuerung des Prozesses vorgegeben wird, sondern dass die KI die Steuerung des Systems / des Prozesses allein aus Beobachtungen lernt.

Wie bereits erwähnt, wird bei dem erfindungsgemäßen Verfahren mindestens ein optimierter Einstellwert für den mindestens einen Wirkparameter computerimplementiert von der Apparatur selbständig anhand mehrerer Bewertungen erlernt, die die Apparatur aus einer jeweiligen Prozessantwort (jeweils in Reaktion auf von der Apparatur vorgegebene Einstellwerte des mindestens einen Wirkparameters), abgeleitet hat. Anhand solcher Einstellwerte des mindestens einen Wirkparameters (also zum Beispiel anhand eines einzustellenden Volumenstroms als einem möglichen Wirkparameter), kann eine jeweilige Steuergröße der Apparatur (also beispielsweise eine Ventilstellung, die den Volumenstrom definiert) angepasst werden. Wie alle anderen Anpassungen kann auch diese vorzugsweise computerimplementiert von der Apparatur selbst (das heißt autonom von menschlichen Eingriffen) vorgenommen werden.

Gemäß einer weiteren möglichen Ausgestaltung ist, insbesondere im Rahmen eines Design-of-Experiments (DoE), vorgesehen, dass die erwähnte künstliche Intelligenz (KI) zunächst Datensätze anlegt, die eine jeweilige gemessene Prozessantwort und einen zugehörigen Satz von Wirkparameterwerten umfasst, wobei diese Wirkparameterwerte gerade die Prozessantwort erzeugen. Die KI kann dann diese Datensätze dazu nutzen, ein Prognosemodell für die Systemantwort aufzustellen und/oder zu optimieren. Dieses Prognosemodell beschreibt somit, wie der Prozess auf bestimmte eingestellte Wirkparameter reagiert. Hierbei muss das Prognosemodell kein explizites Modell sein, was eine genaue Kenntnis des in der Probe / dem System ablaufenden Prozesses voraussetzen würde. Vielmehr kann das Prognosemodell im einfachsten Fall durch Gewichtungen, etwa in einem künstlichen neuronalen Netz, charakterisiert sein, mit Hilfe derer die KI die Prozessantwort in Reaktion auf einen bestimmten Satz aus Wirkparameterwerten prognostiziert. Ein solches Prognosemodell stellt ein zusätzlichen Output dar, der mit dem Verfahren generiert werden kann. Es versteht sich, dass ein solcher Output wertvoll sein kann, um die Steuerung von biologischen, chemischen oder auch physikalischen Prozessen zu optimieren.

Eine besonders bevorzugte Ausgestaltung führt diesen Ansatz weiter, indem die KI mit Hilfe des Prognosemodells virtuelle Systemantworten in Reaktion auf jeweilige virtuelle Sätze von Wirkparameterwerten generiert. Dies bedeutet, dass die KI auf Basis des aus den Messdaten der Prozessantwort gewonnenen Prognosemodells virtuell Systemantworten berechnet, die für einen bestimmten Satz von Wirkparameterwerten erwartet werden kann. Die errechneten virtuellen Systemantworten können danach von der KI anhand der Zielvorgabe klassifiziert werden. Dadurch können solche virtuellen Systemantworten selektiert werden, die von Interessen sind, das heißt, solche Systemantworten, die anzeigen, dass der Prozess in Richtung der Zielvorgabe oder bereits in einem gewünschten Zielzustand abläuft.

Die KI kann dann schließlich die, insbesondere selektierten, virtuellen Systemantworten durch reale Versuche validieren. Hierzu führt die KI mit Hilfe der Apparatur Versuche durch, indem die KI die Apparatur mit einem jeweiligen virtuellen Satz an Wirkparametern steuert und die sich hieraus ergebende reale Prozessantwort misst. Anschließend kann die KI dann die reale Prozessantwort mit der jeweiligen virtuellen Systemantwort vergleichen, die auf Basis des Prognosemodells zuvor von der KI errechnet wurde.

Mit diesem Ansatz lassen sich somit virtuelle Systemantworten identifizieren, die signifikant von dem realen Verhalten des Prozesses abweichen. Daher kann die KI anhand der durchgeführten Validierung, wiederum das Prognosemodell weiter optimieren. Es versteht sich, dass dieser gesamte Optimierungsprozess iterativ wiederholt werden kann. Ein wesentlicher Vorteil dieses Ansatzes, besteht darin, dass die KI nur solche Experimente durchführt, von denen mit hoher Wahrscheinlichkeit angenommen werden kann, dass sie für die Optimierung der Steuerung der Apparatur wertvoll sind. Dadurch werden nicht-sinnvolle Experimente vermieden, was den Zeitaufwand für die Optimierung der Steuerung des Prozesses enorm verkürzt, insbesondere dann, wenn eine große Anzahl an Wirkparametern einzustellen ist und/oder wenn der Prozess eine hohe Komplexität aufweist.

Es kann auch vorgesehen sein, dass neben der Prozessantwort ein Prozesszustand gemessen wird. Bevorzugt werden von der Prozessantwort ein erster Messparameter und von dem Prozesszustand ein zweiter Messparameter gemessen. Hierdurch kann ein zusätzlicher Informationsgewinn erzielt werden, durch den die Steuerung verbessert werden kann.

Weiter kann vorgesehen sein, dass zumindest zwei Messparameter gemessen werden und dass einer der beiden Messparameter verwendet wird, um eine mittels des anderen Messparameters durchgeführte Bewertung zu überprüfen. Bevorzugt wird die Bewertung abhängig von einem Ergebnis der Überprüfung geändert, und zwar besonders bevorzugt von einer KI, insbesondere der zuvor erwähnten KI.

Alternativ können Messergebnisse und/oder Messparameter auch unabhängig voneinander bewertet und von der KI zur Steuerung des Prozesses berücksichtigt werden.

Die Prozessantwort kann insbesondere mindestens zwei unterschiedliche Messparameter umfassen. Es können somit beispielsweise mindestens zwei Messparameter gemessen werden, die beide die Prozessantwort charakterisieren.

Es können allerdings auch zusätzliche Messparameter gemessen werden, die den Zustand des Prozesses betreffen. So kann insbesondere ein erster Messwert den Prozesszustand und ein zweiter Messwert die Prozessantwort betreffen.

Es kann für eine robuste Steuerung vorteilhaft sein, wenn die Messparameter, die die Prozessantwort charakterisieren, ausgewählt sind aus folgender Gruppe von Messparametern: optische Messgrößen, wie etwa ein Absorptionsspektrum, eine emittierte Lichtintensität, oder eine Fluoreszenz; elektrische Größen, insbesondere eine elektrische Leitfähigkeit und/oder Permeabilität und/oder Kapazität; thermische Größen, insbesondere eine Eigenerwärmung einer chemischen oder biologischen Probe, in welcher der Prozess abläuft; pH-Werte.

Die Prozessantwort und/oder der Prozesszustand, insbesondere einzelne Messparameter, können somit optisch und/oder elektrisch und/oder induktiv oder beispielsweise mittels eines pH-Sensors erfasst werden.

Jeder der Messparameter kann anhand einer jeweiligen Zielvorgabe (beziehungsweise einer jeweiligen Zielgröße) mittels einer jeweiligen Bewertung computerimplementiert charakterisiert beziehungsweise bewertet werden. Hierbei kann mindestens ein Einstellwert des mindestens einen Wirkparameters computerimplementiert anhand der Gesamtheit dieser Bewertungen oder einer Auswahl dieser Bewertungen angepasst werden. Bevorzugt kann dann eine künstliche Intelligenz die Bewertungen als Eingabevektor verwenden, insbesondere sodass alle diese Bewertungen bei der Ermittlung des Einstellwerts berücksichtigt werden.

Es kann ferner vorgesehen sein, dass ein erster Messparameter dazu verwendet wird, ein Bewertungskriterium, welches zur Erzeugung einer Bewertung eines zweiten Messparameters verwendet wird, zu überprüfen und/oder anzupassen. In einem solchen Fall ist es vorteilhaft, wenn dies mittels einer Methode des Self-Supervised-Learning erfolgt, bei welchem der erste Messparameter als Grundwahrheit angenommen wird, um die Bewertung des zweiten Messparameters zu verbessern.

Beispielsweise kann ein Absorptionsspektrum als ein erster Messparameter dazu verwendet werden, ein Bewertungskriterium anzupassen, welches bei der optischen Bildanalyse von aufgenommenen Bilddaten, etwa von Zellkulturen, eingesetzt wird. Hierbei können diese Bilddaten dann als ein zweiter Messparameter anhand des angepassten Bewertungskriteriums mittels einer Bewertung charakterisiert werden. Auch ein umgekehrtes Vorgehen ist denkbar.

Anders ausgedrückt, kann eine künstliche Intelligenz anhand eines ersten erfassten Messparameters erlernen, wie ein zweiter Messparameter korrekt zu bewerten ist, um anhand dieser erlernten und damit verbesserten Bewertung eine sinnvolle Anpassung eines Einstellwerts des wenigstens einen Wirkparameters vorzunehmen. Die KI erlernt somit anhand der Erfassung eines ersten Messparameters (der als Label verwendet wird) die korrekte Bewertung eines zweiten Messparameters. Beispielsweise können erfasste Spektren dazu verwendet werden, um Trainingsdaten in Form von zugehörigen Mikroskopbildern zu labeln / zu klassifizieren, um so eine korrekte Bilderkennung gesunder Organismen in den Mikroskopbildern zu erlernen. Dadurch kann auf eine manuelle Klassifikation der Mikroskopbilder verzichtet werden.

Mindestens einer der erfassten Messparameter kann ein globaler Messparameter sein, der von allen der Mikro-Organismen beeinflussbar ist. Also beispielsweise ein Temperaturanstieg in einer biologischen Probe, in welcher der biologische Prozess abläuft, oder ein Transmissionsspektrum eines Kulturmediums, das die Mikro-Organismen ernährt und in welches diese Stoffwechselprodukte abgeben. Dieser globale Messparameter kann insbesondere ohne direkte Messung der Mikro-Organismen erfasst werden, beispielsweise durch eine spektrometrische Messung eines Kulturmediums, wie zuvor erläutert.

Es kann auch vorgesehen sein, dass mindestens einer der erfassten Messparameter ein lokaler Messparameter ist, der nur von einzelnen der Organismen beeinflussbar ist. Ein solcher lokaler Messparameter kann beispielsweise eine bestimmte Anzahl von gesunden Zellen in einem bestimmten Messbereich sein oder eine durchschnittliche Größe einer Auswahl von Zellen. Der lokale Messparameter kann somit insbesondere durch direkte Messung einzelner der Mikro-Organismen erfasst werden, beispielsweise durch eine Bildverarbeitung von Mikroskopbildern der Mikro-Organismen.

Zur Lösung der genannten Aufgabe sind erfindungsgemäß ferner die Merkmale des unabhängigen Vorrichtungsanspruchs vorgesehen. Insbesondere wird somit erfindungsgemäß zur Lösung der Aufgabe bei einer Apparatur der eingangs beschriebenen Art vorgeschlagen, dass die Apparatur folgende Komponenten umfasst: einen Prozessraum zum Aufnehmen eines biologischen, chemischen oder physikalischen Systems, in welchem ein Prozess (insbesondere ein biologischer, chemischer oder physikalischer Prozess wie eingangs beschrieben) abläuft;

einen Controller eingerichtet zum Einstellen mindestens eines Wirkparameters, um den Prozess mittels des Wirkparameters zu steuern; und mindestens ein Messmittel zum Messen einer Prozessantwort des Prozesses, die in Reaktion auf eine Einstellung und/oder Anpassung des mindestens einen Wirkparameters erfolgt. Ferner ist zur Lösung der eingangs genannten Aufgabe vorgesehen, dass durch Messung der Prozessantwort ein Verbrauch und/oder eine Produktion mindestens eines Stoffs durch den Prozess, zumindest indirekt, erfassbar ist und/oder dass durch Messung der Prozessantwort eine Aktivität eines biologischen Systems, welches den Prozess ausführt, erfassbar ist. Hierbei ist der Controller der Apparatur dazu eingerichtet, mindestens einen optimierten Einstellwert für den mindestens einen Wirkparameter computerimplementiert und selbständig anhand mehrerer Bewertungen zu erlernen, wobei die Apparatur die Bewertungen aus einer jeweiligen Prozessantwort, jeweils in Reaktion auf von der Apparatur vorgegebene Einstellwerte des mindestens einen Wirkparameters, abgeleitet hat.

Das mindestens eine Messmittel kann hierbei insbesondere ein Spektrometer und/oder eine Kamera sein. Durch diese Ausgestaltung können mit der Apparatur erfindungsgemäße Verfahren wie zuvor beschrieben oder gemäß einem der auf ein Verfahren gerichteten Ansprüche einfach implementiert werden.

Eine solche Apparatur eignet sich sowohl zum Steuern von biologischen, chemischen wie physikalischen Prozessen.

Der besagte Controller kann also gerade dazu eingerichtet sein, ein erfindungsgemäßes Verfahren wie zuvor beschrieben oder nach einem der auf ein Verfahren gerichteten Ansprüche auszuführen.

Soll die Apparatur für die Steuerung eines biologischen Prozesses verwendet werden, so kann der Prozessraum als ein Kulturraum ausgestaltet sein, der ein Kulturmedium umfasst zum Kultivieren eines biologischen Systems, also beispielsweise von Zellen und/oder Mikro-Organismen. Es kann in diesem Fall ferner bevorzugt vorgesehen sein, dass mindestens eines der Messmittel zur Erfassung eines globalen Messparameters der Prozessantwort eingerichtet ist, der durch alle Elemente des biologischen Systems, insbesondere durch alle Zellen und/oder Mikroorgansimen des Systems, veränderbar ist oder verändert wird.

Ergänzend oder alternativ kann auch vorgesehen sein, dass mindestens eines der Messmittel, insbesondere ein zur Erfassung eines, insbesondere des zuvor beschriebenen, globalen Messparameters eingerichtetes Messmittel, gerade so angeordnet ist, dass die Messung der Prozessantwort zumindest teilweise an einer Messstelle erfolgt, die von dem biologischen System, insbesondere von den Zellen und/oder Mikroorganismen, freigehalten ist. Dadurch kann reproduzierbar eine Veränderung der Umgebungsbedingungen detektiert werden (und zur Anpassung der Steuerung ausgenutzt werden), die auf Stoffwechselvorgänge oder sonstige Veränderungen des biologischen Prozesses zurückzuführen sind.

Bevorzugt kann ferner vorgesehen sein, dass das Messmittel so ausgebildet, angeordnet und/oder eingerichtet ist, dass ein Messwert erfasst wird, der von einer Umgebung von Elementen des biologischen Systems bestimmt ist. Bei der Umgebung kann es sich beispielsweise um das Kulturmedium handeln, in dem das biologische System kultiviert wird.

Eine weitere bevorzugte Ausgestaltung, die sich insbesondere zur Steuerung von Zellen und/oder mikro-biologischer Prozesse aber auch von chemischen oder physikalischen Prozessen eignet, sieht vor, dass die Apparatur eine mikrofluidische Vorrichtung zur Versorgung des Systems mit einem Medium, insbesondere einer Flüssigkeit oder dem zuvor beschriebenen Kulturmedium, umfasst. Diese Vorrichtung kann beispielsweise eine Vielzahl an Kammern oder Gefäßen aufweisen, in denen jeweils biologische Proben aufgenommen werden können. Die Vorrichtung kann ferner auch als eine Durchflussvorrichtung ausgestaltet sein, sodass das Kulturmedium in Form einer Flüssigkeit an den Elementen des biologischen Systems vorbeiströmen kann.

Es kann ferner vorgesehen sein, dass die Vorrichtung eine mikrofluidisch wirksame Trennstruktur aufweist, mit der das biologische System, insbesondere die Mikroorganismen, von einer, insbesondere der zuvor beschriebenen, Messstelle fernhaltbar sind, an welcher mit dem mindestens einen Messmittel die Prozessantwort erfassbar ist. Mögliche Trennstrukturen sind beispielsweise eine Membran, ein Filter, oder mikroskopisch dimensionierte Durchflussöffnungen, durch die die Mikro-Organismen aufgehalten werden können.

Schließlich kann das mindestens eine Messmittel, zumindest teilweise, in die mikrofluidische Vorrichtung integriert sein. In diesem Fall kann somit die Messstelle unveränderlich in Bezug auf die Trennstruktur sein, was zu einer reproduzierbaren und genauen Erfassung der Prozessantwort führt. Aber auch bei Verzicht auf eine Trennstruktur, kann die Integration Sinn machen, um die Robustheit der Messung zu erhöhen.

Die Apparatur kann ferner mindestens ein Stellglied aufweisen, wie etwa ein ansteuerbares Ventil, mit der der mindestens eine Wirkparameter veränderbar ist und wobei das mindestens eine Stellglied mittels des Controllers regelbar ist.

Es kann ferner vorgesehen sein, dass der Controller dazu eingerichtet ist, mindestens einen Einstellwert des mindestens einen Wirkparameters computerimplementiert anhand einer Bewertung der Prozessantwort anzupassen, und zwar insbesondere durch Vergleich mit einer Zielvorgabe für die Prozessantwort. Hierdurch kann der Controller beispielsweise die Prozessantwort an die Zielvorgabe heranführen.

Schließlich sei noch erwähnt, dass die Apparatur selbstverständlich zur Ausführung eines der zuvor erläuterten Verfahren eingerichtet sein kann und/oder über die zur Ausführung eines der zuvor erläuterten Verfahren notwendigen Mittel (Messmittel, Regelmittel, elektronische Speicher, Probennahme-Vorrichtung etc.) verfügen kann.

Die Erfindung wird nun anhand von Ausführungsbeispielen näher beschrieben, ist aber nicht auf diese Ausführungsbeispiele beschränkt. Weitere Ausbildungen der Erfindung können aus der nachfolgenden Beschreibung eines bevorzugten Ausführungsbeispiels in Verbindung mit der allgemeinen Beschreibung, den Ansprüchen sowie den Zeichnungen gewonnen werden.

Bei der folgenden Beschreibung verschiedener bevorzugter Ausführungsformen der Erfindung erhalten in ihrer Funktion übereinstimmende Elemente auch bei abweichender Gestaltung oder Formgebung übereinstimmende Bezugszahlen. Das vorgestellte Beispiel betrifft die Steuerung eines biologischen Prozesses. Das erfindungsgemäße Verfahren und die zugehörige Vorrichtung sind aber für den Fachmann anhand der Darlegungen auch auf chemische oder physikalische Systeme und Prozesse übertragbar.

Es zeigt:

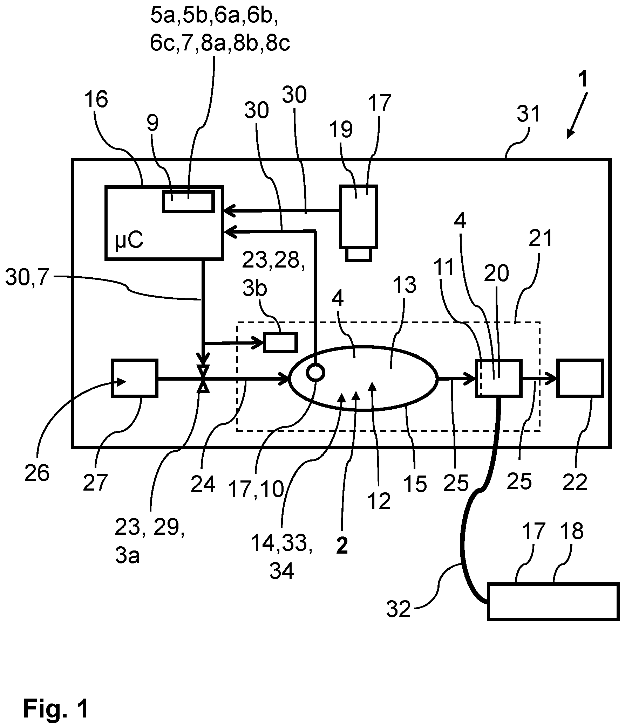

- Fig. 1: eine schematische Übersicht einer erfindungsgemäßen Apparatur zum Steuern eines biologischen Prozesses, der in der Apparatur abläuft.

Die Figur 1 zeigt eine im Ganzen mit 1 bezeichnete Apparatur mit der sich ein biologischer Prozess 2, der in der Apparatur 1 abläuft, autonom, das heißt ohne menschlichen Eingriff von außen, steuern lässt. Die Apparatur 1 umfasst einen Kulturraum 15 in Form einer mikrofluidischen Kammer, durch die ein Kulturmedium 13 leitbar ist, welches ein biologisches System 34 ernährt, welches in dem Kulturraum 15 kultiviert wird. Das biologische System 34 kann insbesondere aus Elementen 14 bestehen oder solche aufweisen, wobei die Elemente Zellen 14 und/oder Mikroorganismen 14 sind. Der biologische Prozess 2 läuft somit in der mikrofluidischen biologischen Probe 12 ab, die durch das biologische System 34 und das Kulturmedium 13 in dem Kulturraum 15 gebildet ist.

Die Apparatur 1 weist ferner ein Reservoir 27 auf, in dem mehrere Stoffe 26 bevorratet sind, die über die gezeigte Zuleitung 24 (die mehrere separate Leitungen umfasst) in den Kulturraum 15 leitbar ist, um so den dort ablaufenden biologischen Prozess 2 als jeweiliger Wirkparameter 3a zu beeinflussen. Der eigentliche Wirkparameter 3a stellt dabei die Flussrate dar, mit welcher ein bestimmter der Stoffe 26 in den Kulturraum 15 geleitet wird. Die bedeutet, dass es mehrere Wirkparameter 3a gibt, die jeweils eine Flussrate eines der Stoffe 26 von dem Reservoir 27 in den Kulturraum 15 charakterisiert.

Um diesen Wirkparameter 3a einzustellen, verfügt die Apparatur 1 über mindestens ein Stellglied 23 in Form eines Ventils 29, welches über eine Datenleitung 30 mit einem Controller 16 verbunden ist. Der Controller 16 kann somit steuernd auf das verstellbare Ventil 29 zugreifen und dadurch die Durchflussrate, das heißt den Wirkparameter 3a, regeln beziehungsweise einstellen.

Die Apparatur 1 umfasst ferner ein weiteres Stellglied 23 in Form eines Heizelements 28, mit welchem sich eine globale Temperatur, die in dem Kulturraum 15 herrscht, verändern lässt. Die Temperatur in dem Kulturraum 15 kann dabei durch den Controller 16 mittels des gezeigten Temperatursensors 10 überwacht werden, der über eine Datenleitung 30 mit dem Controller 16 verbunden ist. Ferner kann der Controller 16 auch über die gezeigte Datenleitung 30 auf das Heizelement 28 steuernd einwirken (durch Vorgabe eines elektrischen Heizstroms) und dadurch eine Heizleistung als einen weiteren Wirkparameter 3b einstellen, die dem Kulturraum 15 und damit dem biologischen Prozess 2 mittels des Heizelements 28 zuführbar ist.

Die Apparatur 1 weist ferner eine Kamera 19 als Messmittel 17 auf, mit der sich Mikroskopbilder der biologischen Probe, insbesondere des biologischen Systems 34 wie etwa von Zellen einer Zellkultur 33 aufzeichnen lassen, die in dem Kulturraum 15 heranwachsen.

Über die Ableitung 25 kann das Kulturmedium 13 in regelmäßigen Abständen abgelassen werden und zu der gezeigten Messstelle 20 transportiert werden. An der Messstelle 20 ist ein weiteres Messmittel 17 in Form eines Spektrometers 18 über eine Glasfaser 32 verbunden und kann somit an der Messstelle 20 das Kulturmedium 13 spektroskopisch vermessen. Hierbei sorgt eine mikrofluidisch wirksame Trennstruktur 11 in Form eines Filters dafür, dass die Elemente des biologischen Systems 34, die in dem Kulturraum 15 kultiviert werden, von der Messstelle 20 ferngehalten sind, so dass die spektrometrische Messung, die mit dem Spektrometer 18 durchgeführt wird, unter Ausschluss des biologischen Systems 34 erfolgt, wobei hierzu nur das Kulturmedium 13 nicht jedoch die darin befindlichen Elemente 14 des biologischen Systems 34 durchleuchtet werden. In anderen Anwendungen kann der Filter auch entfernt werden, sodass dann das Spektrometer 18 sowohl das Kulturmedium 13 als auch die darin befindlichen Elemente 14 spektral vermisst. Dadurch ließe sich beispielsweise eine OD600-Messung realisieren (Vgl. weiter oben).

Nachdem das Kulturmedium 13 die Messstelle 20 passiert hat, gelangt es über die weitere Ableitung 25 in einen Auffangbehälter 22.

Wie durch die gestrichelte Linie in Figur 1 angedeutet ist, ist ein Teil der Apparatur 1 als eine mikrofluidische Vorrichtung 21 ausgestaltet. Diese dient zum einen der Versorgung des biologischen Systems 34 mit dem Kulturmedium 13; zum anderen der Aufnahme von Mikroskopbildern mit der Kamera 19 und schließlich der zuvor erläuterten spektrometrischen Messung. Hierfür ist die Glasfaser 32 in die mikrofluidische Vorrichtung 21 integriert, während die eigentliche Messanordnung des Spektrometers 18 außerhalb eines Gehäuses 31 der Apparatur 1 angeordnet ist, die die sonstigen Komponenten der Apparatur 1 aufnimmt.

Auch das Spektrometer 18 wird von dem Controller 16 gesteuert und ausgelesen. Mit der spektrometrischen Messung kann zudem indirekt erfasst werden, welche chemischen Substanzen oder biologischen Organismen von dem biologischen Prozess 2 erzeugt werden (was als Chemo- oder Biogenese bekannt ist).

Durch die spektrometrische Messung kann somit indirekt sowohl ein Verbrauch von bestimmten Stoffen als auch die Produktion von bestimmten Stoffen durch den biologischen Prozess 2 erfasst werden. Diese Erfassung geschieht gerade während der biologische Prozess 2 abläuft und während - durch Einstellen der Wirkparameter 3 - Einfluss auf den biologischen Prozess 2 genommen wird.

Der Controller 16 der Apparatur 1 ist nun gerade zum Einstellen der beiden Wirkparameter 3a, 3b eingerichtet, also des Durchflusses 3a, mit dem der Stoff 26 in den Kulturraum 15 gelangt sowie der Heizleistung 3b, die mittels des Heizelements 28 dem Kulturraum 15 und damit dem biologischen Prozess 2 zugeführt wird. Durch das Einstellen der Wirkparameter 3 nimmt der Controller 16 Einfluss auf Bedingungen, die den biologischen Prozess 2 beeinflussen, wodurch der biologische Prozess 2 gesteuert werden kann.

Der Mikrocontroller 16 weist ferner einen Speicher 9 auf, in dem eine Zielvorgabe 5 in Form eines Zielspektrums abgelegt ist.

In Reaktion auf die von dem Controller 16 eingestellten Wirkparameter 3a, 3b verändert sich der biologische Prozess 2, wobei sich dadurch eine Stoffwechselaktivität der Mikro-Organismen 14 verändert. Dadurch erhöht sich zum einen ein Stoffwechselverbrauch; zum anderen produzieren die Mikro-Organismen 14 aber auch Stoffwechselprodukte, die sie in das Kulturmedium 13 abgeben. Mit anderen Worten reagiert der biologische Prozess 2 beziehungsweise das für diesen verantwortliche biologische System 34 je nach eingestellten Wirkparametern 3a, 3b mit einer bestimmten Prozessantwort 4, die der biologische Prozess 2 in Reaktion auf eine Anpassung von wenigstens einem der beiden Wirkparameter 3a, 3b auf seine unmittelbare Umgebung, nämlich das Kulturmedium 13, überträgt.

Diese Prozessantwort 4 wird zum einen spektrometrisch mittels des Spektrometers 18 von dem Controller 16 gemessen, wobei das an der Messstelle 20 von dem Kulturmedium 13 gemessene Spektrum als ein Messparameter 8a der Prozessantwort 4 in einem internen Speicher 9 des Controllers 16 abgelegt wird.

Mittels des Temperatursensors 10 kann der Controller 16 ferner einen Temperaturanstieg der biologischen Probe 12, die in dem Kulturraum 15 durch das Kulturmedium 13 und das biologische System 34 gebildet ist, als einen zweiten Messparameter 8b der Prozessantwort 4 messen. Dieser Temperaturanstieg 8b wird ebenfalls als ein weiterer Messparameter 8 und damit als eine weitere Komponente der Prozessantwort 4 in dem Speicher 9 abgelegt. Der Temperaturanstieg wird dabei zu Zeitpunkten gemessen, in denen gerade keine Heizleistung zugeführt wird, sodass der Temperaturanstieg 8b auf den biologischen Prozess 2 zurückgeführt werden kann, das heißt von diesem erzeugt wird (Thermogenese). Auch dieser zweite Messparameter 8b wird in dem Speicher 9 abgelegt und kann somit von dem Controller 16 weiter verarbeitet werden.

Durch den biologischen Prozess 2, der durch das biologische System 34 befeuert wird und in dem Kulturraum 15 abläuft, werden zudem zahlreiche Umgebungsfaktoren verändert, nämlich etwa die stoffliche Zusammensetzung des Kulturmediums 13, dessen Temperatur, dessen PH-Wert, die elektrische Leitfähigkeit des Kulturmediums 13 und auch sein optisches Transmissionsverhalten. Alle diese Umgebungsfaktoren können gemäß dem erfindungsgemäßen Verfahren grundsätzlich als einzelne Messparameter 8 der Prozessantwort 4 des biologischen Prozesses 2 gemessen werden, um so jeweilige Änderungen dieser Umgebungsfaktoren in Reaktion auf die jeweilige Anpassung eines der Wirkparameter 3 zu erfassen.

In einem nächsten Schritt vergleicht der Controller 16 die beiden gemessenen Messparameter 8a, 8b mit einer jeweiligen Zielvorgabe 5, also einmal einem Zielspektrum 5a (als erster Zielgröße) und einmal einem Zielwert für den Temperaturanstieg 5b (als zweiter Zielgröße). Anschließend wird jeder der als Prozessantwort 4 erfassten beiden Messparameter 8a und 8b anhand der jeweiligen Zielvorgabe 5a, 5b mittels einer jeweiligen Bewertung 6a, 6b computerimplementiert von dem Controller 16 bewertet. Anschließend passt der Controller 16 entweder den Wirkparameter 3a, oder den Wirkparameters 3b oder aber beide Wirkparameter 3a, 3b computerimplementiert anhand dieser beiden Bewertungen 6a, 6b an.

Diese Anpassung der Wirkparameter 3a, 3b erfolgt dabei mit dem Ziel, die gemessene Prozessantwort 4 näher an die in dem Speicher 9 als Referenzantworten hinterlegten Zielvorgaben heranzuführen, das heißt den biologischen Prozess 2 so zu steuern, dass eine gewünschte Temperaturerhöhung und ein bestimmtes Spektrum als Prozessantwort 4 erhalten wird. Im Unterschied zur Regelung der globalen Temperatur in dem Kulturraum 13 mittels des Heizelements 28 wird dabei nicht etwa gemessen, wie eine zugeführte Heizleistung die Temperatur in dem Kulturraum 15 verändert, sondern es wird mit dem Temperatursensor 10 erfasst, welche Wärmemenge durch die Stoffwechselaktivität des biologischen Systems 34 als Teil der Prozessantwort 4 produziert wird, was unter dem Begriff der Thermogenese bekannt ist.

Es versteht sich, dass sich dieser Ansatz etwa auch auf die Detektion einer Fotogenese, also die Produktion von Licht durch eine Stoffwechselaktivität des biologischen Systems 34, ausweiten lässt, wobei dann mittels eines photosensitiven Sensors das von dem biologischen Prozess 2 produzierte Licht gemessen würde. Auch dies unterscheidet sich beispielsweise von der Messung einer Lichtintensität, die mittels einer Lichtquelle - zum Beispiel als ein weiterer möglicher Wirkparameter 3 - dem Kulturraum 15 zugeführt wird, etwa um dort eine Fotosynthese mit Hilfe von Mikro-Organismen 14 auszulösen.

Der Controller 16, der als Mikrocontroller ausgebildet sein kann, erlernt dabei die vorzunehmende Anpassung von Einstellwerten der beiden Wirkparameter 3b und 3c mittels einer künstlichen Intelligenz, die in Form eines künstlichen neuronalen Netzes (KNN) realisiert ist. Mit anderen Worten basiert die Anpassung des Wirkparameters 3 als auch die Bewertung der jeweiligen gemessenen Messparameter 8a, 8b auf einer Methode des maschinellen Lernens.

Durch das maschinelle Lernen wird es möglich, dass der Controller 16 selbständig und computerimplementiert anhand von mehreren Bewertungen 6 erlernt, wie die einzelnen Wirkparameter 3 so eingestellt werden, dass sich die gemessene Prozessantwort 4 immer näher annähert an eine gewünschte Prozessantwort 4, die gerade einen Zustand des biologischen Prozesses 2 beschreibt, der durch Steuerung mittels der Apparatur 1 erreicht werden soll.

Aus diesen Erläuterungen wird somit klar, dass der Controller 16 mittels des Temperatursensors 10 und des Spektrometers 18 zwei Messparameter 8, nämlich das gemessene Spektrum 8a und eine endogene Temperaturerhöhung 8b innerhalb der biologischen Probe 12 misst, anschließend die aus diesen zwei Messparametern 8a, 8b bestehende Prozessantwort 4 mittels einer jeweiligen Bewertung 6a, 6b computerimplementiert bewertet und anschließend computerimplementiert anhand der Bewertung 6 die jeweiligen Einstellwerte der Wirkparameter 3a, 3b anpasst.

Die Anpassung der Wirkparameter 3a geschieht nun dadurch, dass der Controller 16 einerseits durch Ansteuerung des Ventils 29 die Menge des Stoffs 26 regelt, die aus dem Reservoir 27 entnommen und dem biologischen Prozess 2 in dem Kulturmedium 13 zugeführt wird. Zum anderen kann der Controller 16 den Wirkparameter 3b, also die Heizleistung, dadurch einstellen, dass er mehr oder weniger Steuerstrom an das Heizelement 28 sendet, sodass dieses dem Kulturraum 15 mehr oder weniger Wärme zuführt. Das heißt der Controller 16 kann den Temperatursensor 10 einmal als Messmittel 17 verwenden, um den Messparameter 8b der Thermogenese, die von dem biologischen Prozess 2 erzeugt wird, zu vermessen. Zum anderen kann der Controller 16 den Temperatursensor 10 aber auch dazu einsetzen, die mittels des Heizelements 28 erzeugte Heizleistung 3b zu regeln. Es versteht sich, dass der Temperatursensor 10 für diese beiden Aufgaben insbesondere wechselweise eingesetzt werden kann, etwa in vorab festgelegten zeitlichen Intervallen der Prozessführung.

Wie bereits erläutert wurde, erfolgt die Bewertung der Prozessantwort 4 anhand von jeweiligen Zielvorgaben 5a, 5b für die einzelnen Messparameter 8 der Prozessantwort 4. Die beiden bislang erläuterten Messparameter 8a und 8b, nämlich das gemessene Spektrum 8a und der endogene Temperaturanstieg 8b in der biologischen Probe 12, können jeweils als globale Messparameter aufgefasst werden, da diese von allen Mikro-Organismen 14, die sich in dem Kulturmedium 13 befinden, beeinflusst werden. Hierbei wird zumindest das Spektrum 8a als globaler Messparameter ohne eine direkte Messung der Mikro-Organismen 14 erfasst.

Mittels der gezeigten Kamera 19 lässt sich zudem ein weiterer Messparameter 8 erfassen. Bei diesem kann es sich insbesondere auch um einen Messparameter 8 eines Zustands des biologischen Prozesses handeln wie etwa um die Anzahl an Zellen 8c, die innerhalb eines vorgegebenen Sichtfensters der mikrofluidischen Vorrichtung 21 mittels einer Bildverarbeitung von der Kamera 19 erfasst werden können, zu einem bestimmten Zeitpunkt. Die Erfindung schlägt nun gerade in einer Variante vor, zusätzlich einen solchen Messparameter über einen Prozesszustands anhand einer Zielvorgabe 5 zu bewerten und anschließend anhand dieser Bewertung die Anpassung von mindestens einem der bereits erläuterten Wirkparameter 3 vorzunehmen.

Der Parameter 8c kann zudem als ein lokaler Parameter aufgefasst werden, da dieser nur von einzelnen Elementen des biologischen Systems 34 beeinflussbar ist. Beispielsweise hat die Zunahme von Mikro-Organismen 14 außerhalb des Sichtfensters keinerlei Einfluss auf die Messung der Anzahl 8c an Zellen innerhalb des Sichtfensters. Ferner unterscheidet sich dieser lokale Messparameter 8c von den beiden anderen Messparametern 8a und 8b dadurch, dass dieser mittels einer direkten Messung einzelner Elemente des biologischen Systems 34 erfasst wird, nämlich durch eine Bildverarbeitung von Mikroskopbildern dieser Elemente, die mit der Kamera 19 aufgenommen werden.

Da das gemessene Spektrum als auch die Mikroskopbilder gleichzeitig erfasst werden, und somit jeweils einen momentanen Zustand des biologischen Prozesses 2 beschreiben, kann das gemessene Spektrum als ein erster Messparameter 8a der erfassten Prozessantwort 4 dazu verwendet werden, ein Bewertungskriterium, welches benutzt wird, um eine Bewertung 6c der als weiteren Messparameter 8c verwendeten Anzahl an Zellen pro Mikroskopbild zu erzeugen, entsprechend so anzupassen, dass eine sinnvolle Bewertung der aufgezeichneten Mikroskopbilder erfolgen kann. Mit anderen Worten wird also vorgeschlagen, insbesondere durch Verwendung einer Methode des Self-Supervised-Learning, das aufgenommene Spektrum 8a als eine Grundwahrheit zu betrachten, und die erlernte Bewertung 6a des Spektrums (die anhand des im Speicher 9 hinterlegten Zielspektrum erfolgt) dazu zu benutzen, die Bewertung 6c der Anzahl 8c an Zellen in den einzelnen Mikroskopbilder zu verbessern. Vereinfacht gesprochen lernt die Apparatur somit selbstständig, welche Anzahl an Zellen in einem bestimmten Zustand des biologischen Prozesses 2 vernünftigerweise erwartet werden können beziehungsweise erzielbar sind und zwar anhand der erlernten Bewertung des gemessenen Spektrums 8a, welches einen Rückschluss zulässt auf den momentanen Zustand des biologischen Systems 34 / des biologischen Prozesses 2.

Umgekehrt können aber auch die Mikroskopbilder, genauer der daraus abgeleitete Messparameter einer bestimmten Zelldichte, dafür verwendet werden, eine KI zu trainieren, aufgenommene Spektren 8a korrekt (das heißt sinnvoll) zu bewerten. Allgemein kann also insbesondere vorgesehen sein, dass ein Messparameter, der einen Zustand des biologischen Prozesses 2 beschreibt, von einer, insbesondere der zuvor erwähnten, KI dazu verwendet wird, eine Bewertung 6a eines Messparameters 8a der Prozessantwort 4 zu erlenen.

Zusammenfassend wird zur verbesserten autonomen Steuerung eines Prozesses 2 mittels einer Apparatur 1 durch Einstellen von mindestens einem den Prozess 2 steuernden Wirkparameter 3 vorgeschlagen, dass durch ein Messmittel 17, vorzugsweise ein in die Apparatur 1 integriertes Spektrometer 18, eine Prozessantwort 4, die der Prozess 2 in Reaktion auf eine Anpassung des wenigstens einen Wirkparameters 3 auf seine unmittelbare Umgebung überträgt, gemessen und computerimplementiert, vorzugsweise unter Verwendung einer künstlichen Intelligenz, bewertet wird und dass anhand dieser Bewertung der mindestens eine Wirkparameter 3 von der Apparatur 1 automatisiert nachjustiert wird, etwa um den Prozess 2 in eine gewünschte Richtung zu steuern. Dieser Ansatz ist sowohl auf biologische, chemische wie physikalische Prozesse anwendbar.

### Bezugszeichenliste

- 1: Apparatur (z.B. ausgestaltet als Inkubator)
- 2: biologischer/chemischer/physikalischer Prozess (der in 1, genauer in 15 abläuft)
- 3: Wirkparameter (zur Steuerung von 2)
- 4: Prozessantwort (von 2)
- 5: Zielvorgabe (target)
- 6: Bewertung (von 4)
- 7: Steuergröße (zur Beeinflussung von 3)
- 8: Messparameter (als Teil von 4)
- 9: Speicher
- 10: Temperatursensor
- 11: Trennstruktur (z.B. Membran, Filter, Durchflussöffnung)
- 12: Probe (insbesondere biologische Probe)
- 13: Medium, insbesondere Kulturmedium
- 14: Elemente von 34 (z.B. Zellen, Mikroorganismen, Bakterien, Algen, etc.)
- 15: Prozessraum (insbesondere Kulturraum zum Kultivieren einer biologischen Probe)
- 16: Controller
- 17: Messmittel
- 18: Spektrometer
- 19: Kamera
- 20: Messstelle
- 21: mikrofluidische Vorrichtung (z.B. mikrofluidischer Chip)
- 22: Auffangbehälter
- 23: Stellglied
- 24: Zuleitung
- 25: Ableitung
- 26: Stoff
- 27: Reservoir (für 26)
- 28: Heizelement
- 29: Ventil
- 30: Datenleitung
- 31: Gehäuse
- 32: Glasfaser
- 33: Zellkultur
- 34: System (insbesondere biologisches System)

## Patentansprüche

1. **Verfahren zum Steuern einer Apparatur (1),** in der ein Prozess (2) abläuft,
- wobei der Prozess (2) durch Einstellen mindestens eines Wirkparameters (3) gesteuert wird,
**dadurch gekennzeichnet,**
- **dass** eine Prozessantwort (4) des Prozesses (2) auf den mindestens einen Wirkparameter (3) gemessen wird,
- wobei durch Messung der Prozessantwort (4)
- ein Verbrauch und/oder eine Produktion mindestens eines Stoffs durch den Prozess (2), zumindest indirekt, erfasst wird und/oder
- eine Aktivität eines biologischen Systems (34), welches den Prozess ausführt, erfasst wird,
- **dass** die gemessene Prozessantwort (4) mittels einer Bewertung (6) computerimplementiert anhand einer vorgegebenen Zielvorgabe (5) für die gemessene Prozessantwort (4) bewertet wird, und
- **dass** mindestens ein Einstellwert des mindestens einen Wirkparameters (3) computerimplementiert anhand der Bewertung (6) angepasst wird,
- wobei mindestens ein optimierter Einstellwert für den mindestens einen Wirkparameter (3) computerimplementiert von der Apparatur (1) selbständig anhand mehrerer Bewertungen (6) erlernt wird, die die Apparatur (1) aus einer jeweiligen Prozessantwort (4), jeweils in Reaktion auf von der Apparatur (1) vorgegebene Einstellwerte des mindestens einen Wirkparameters (3), abgeleitet hat.

2. Verfahren gemäß Anspruch 1, wobei die Zielvorgabe (5) eine Referenzantwort (5) für die Prozessantwort (4) ist,
- um so die Prozessantwort (4) an die Zielvorgabe (5) anzugleichen.

3. Verfahren gemäß Anspruch 1 oder Anspruch 2, wobei der Prozess (2) in einer Probe (12) abläuft, die ein Kulturmedium (13) umfasst und die Prozessantwort (4) in dem Kulturmedium (13) gemessen wird, insbesondere unter Ausschluss von Zellen und/oder Mikroorganismen der biologischen Probe (12).

4. Verfahren gemäß einem der vorhergehenden Ansprüche, wobei die Prozessantwort (4) des Prozesses (2) auf ein Medium (13) gemessen wird, das Elemente (14) des dem Prozess (2) zugrundeliegenden Systems (34) umgibt, insbesondere wobei das System (34) in das ein Kulturmedium (13) bildende Medium (13) eingebettet ist.

5. Verfahren gemäß einem der vorhergehenden Ansprüche, wobei mindestens ein Messparameter (8) der Prozessantwort (4) in einer direkten Umgebung der Elemente (14) des dem Prozess (2) zugrundeliegenden biologischen Systems (34) gemessen wird, jedoch ohne direkte Messung der Elemente (14) des biologischen Systems (34).

6. Verfahren gemäß einem der vorhergehenden Ansprüche, wobei der Verbrauch und/oder die Produktion mindestens eines Stoffs durch den Prozess (2) erfasst wird, während auf den Prozess (2) durch Einstellen des mindestens einen Wirkparameters (3) Einfluss genommen wird.

7. Verfahren gemäß einem der vorhergehenden Ansprüche, wobei die durch Messung der Prozessantwort (4) erfasste Aktivität des biologischen Systems (34)
- eine Thermogenese, wie z.B. die Produktion von Wärme durch Stoffwechselaktivität, und/oder
- eine Chemo- oder Biogenese, wie z. B. die Produktion chemischer Substanzen oder biologischer Organismen durch Stoffwechselaktivität, und/oder
- eine Photogenese, wie z. die Produktion von Licht durch Stoffwechselaktivität, und/oder
- ein Energieverbrauch und/oder
- ein Stoffverbrauch oder eine Stoffproduktion ist.

8. Verfahren gemäß einem der vorhergehenden Ansprüche, wobei der Prozess (2) mindestens einen Umgebungsfaktor verändert, beispielsweise
- eine stoffliche Zusammensetzung und/oder
- Temperatur und/oder
- ph-Wert und/oder
- Permittivität und/oder
- elektrische Leitfähigkeit und/oder
- ein optisches Transmissions- oder Reflektionsverhalten und/oder
- ein Umgebungsfaktor eines Kulturmediums (13), in welchem das biologische System (34) kultiviert wird,
und wobei der mindestens eine Umgebungsfaktor als mindestens ein Messparameter (8) der Prozessantwort (4) gemessen wird, um eine Änderung des mindestens einen Umgebungsfaktors in Reaktion auf die Anpassung des mindestens einen Wirkparameters (3) zu erfassen.

9. Verfahren gemäß einem der vorhergehenden Ansprüche, wobei wenigstens ein Messparameter (8a) der Prozessantwort (4) durch eine spektrometrische Messung,
- insbesondere eines Kulturmediums (13), in welchem das biologische System (34) kultiviert wird,
erfasst wird.

10. Verfahren gemäß Anspruch 9, wobei hierzu nur das Kulturmedium (13) nicht jedoch die darin befindlichen Elemente (14) des biologischen Systems (34) durchleuchtet werden oder sowohl das Kulturmedium (13) als auch die darin befindlichen Elemente (14).

11. Verfahren gemäß einem der vorhergehenden Ansprüche, wobei die Anpassung des jeweiligen Einstellwerts des mindestens einen Wirkparameters (3) mittels einer künstlichen Intelligenz computerimplementiert erfolgt, insbesondere mittels einer Methode des maschinellen Lernens.

12. Verfahren gemäß Anspruch 11,
- wobei die künstliche Intelligenz Datensätze umfassend
- eine jeweilige gemessene Prozessantwort (4) und
- einen zugehörigen Satz von diese Prozessantwort (4) erzeugenden Wirkparameterwerten
dazu nutzt, ein Prognosemodell für die Systemantwort (4) aufzustellen und/oder zu optimieren,
- vorzugsweise wobei die künstlichen Intelligenz mit Hilfe des Prognosemodells virtuelle Systemantworten in Reaktion auf jeweilige virtuelle Sätze von Wirkparameterwerten generiert.

13. Verfahren gemäß Anspruch 12,
- wobei die künstliche Intelligenz die virtuellen Systemantworten, insbesondere nach Selektion anhand der Zielvorgabe (5), durch reale Versuche validiert,
- vorzugsweise indem die künstlichen Intelligenz
- die Apparatur (1) mit dem jeweiligen virtuellen Satz an Wirkparametern steuert,
- die sich hieraus ergebende reale Prozessantwort (4) misst und
- mit der jeweiligen zuvor errechneten virtuellen Systemantwort vergleicht.

14. Verfahren gemäß einem der Ansprüche 11 bis 13, wobei der mindestens eine optimierter Einstellwert für den mindestens einen Wirkparameter (3) computerimplementiert von der künstlichen Intelligenz selbständig anhand der mehreren Bewertungen (6) erlernt wird.

15. Verfahren gemäß einem der vorhergehenden Ansprüche, wobei neben der Prozessantwort (4) ein Prozesszustand gemessen wird, insbesondere wobei von der Prozessantwort (4) ein erster Messparameter (8) und von dem Prozesszustand ein zweiter Messparameter (8) gemessen werden.

16. Verfahren gemäß einem der vorhergehenden Ansprüche, wobei zumindest zwei Messparameter (8) gemessen werden und wobei einer der beiden Messparameter (8) verwendet wird, um eine mittels des anderen Messparameters (8) durchgeführte Bewertung zu überprüfen,
- insbesondere wobei die Bewertung abhängig von einem Ergebnis der Überprüfung geändert wird, vorzugsweise von einer, insbesondere der, künstlichen Intelligenz.

17. **Apparatur (1) zum autonomen Steuern eines Prozesses (2),** beispielsweise eines Wachstums oder eines Stoffwechsels eines biologischen Systems (34) oder beispielsweise wobei der Prozess (2) ein chemischer oder ein physikalischer Prozess ist, wobei die Apparatur (1) umfasst:
- einen Prozessraum (15) zum Aufnehmen eines biologischen, chemischen oder physikalischen Systems, in welchem der Prozess (2) abläuft,
- einen Controller (16) eingerichtet zum Einstellen mindestens eines Wirkparameters (3), um den Prozess (2) mittels des Wirkparameters (3) zu steuern, und
- mindestens ein Messmittel (17), insbesondere
- ein Spektrometer (18) und/oder
- eine Kamera (19),
zum Messen einer Prozessantwort (4) des Prozesses (2), die in Reaktion auf eine Anpassung des mindestens einen Wirkparameters (3) erfolgt, **dadurch gekennzeichnet,**
- **dass** durch Messung der Prozessantwort (4)
- ein Verbrauch und/oder eine Produktion mindestens eines Stoffs durch den Prozess (2), zumindest indirekt, erfassbar ist und/oder
- eine Aktivität eines biologischen Systems (34), welches den Prozess ausführt, erfassbar ist, und
- **dass** der Controller (16) dazu eingerichtet ist, mindestens einen optimierten Einstellwert für den mindestens einen Wirkparameter (3) computerimplementiert und selbständig anhand mehrerer Bewertungen (6) zu erlernen, wobei die Apparatur (1) die Bewertungen (6) aus einer jeweiligen Prozessantwort (4), jeweils in Reaktion auf von der Apparatur (1) vorgegebene Einstellwerte des mindestens einen Wirkparameters (3), abgeleitet hat.

18. Apparatur (1) gemäß dem vorhergehenden Anspruch und eingerichtet zur Steuerung eines biologischen Prozesses (2), wobei mindestens eines der Messmittel (17) zur Erfassung eines globalen Messparameters (8) der Prozessantwort (4) eingerichtet ist, der durch alle Elemente (14) des biologischen Systems (34), das dem Prozess (2) zugrunde liegt, veränderbar ist oder verändert wird.

19. Apparatur gemäß Anspruch 17 oder 18, wobei mindestens eines der Messmittel (17),
- insbesondere ein zur Erfassung des globalen Messparameters (8) eingerichtetes Messmittel (17),
so angeordnet ist, dass die Messung der Prozessantwort (4) zumindest teilweise an einer Messstelle (20) erfolgt, die von den Elementen (14) des biologischen Systems (34) freigehalten ist.

20. Apparatur (1) gemäß einem der Ansprüche 17 bis 19, wobei die Apparatur (1) eine mikrofluidische Vorrichtung (21) zur Versorgung des Systems (34) mit einem Medium, insbesondere einem Kulturmedium (13), umfasst,
- vorzugsweise wobei die Vorrichtung (21) eine mikrofluidisch wirksame Trennstruktur (22) aufweist, mit der die Elemente (14) des biologischen Systems (34) von einer, insbesondere der in Anspruch 19 erwähnten, Messstelle (20) fernhaltbar sind, an welcher mit dem mindestens einen Messmittel (17) die Prozessantwort (4) erfassbar ist.

## Claims

1. **Method for controlling an apparatus (1),** in which a process (2) takes place,
- wherein the process (2) is controlled by setting at least one action parameter (3),
**characterized in that**
- a process response (4) of the process (2) on the at least one action parameter (3) is measured,
- wherein, by measuring the process response (4),
- a consumption and/or a production of at least one material by the process (2) is, at least indirectly, acquired and/or
- an activity of a biological system (34), which carries out the process, is acquired,
- the measured process response (4) is evaluated by means of an evaluation (6) in a computer-implemented manner based on a predetermined preset target value (5) for the measured process response (4), and
- at least one set value of the at least one action parameter (3) is adjusted in a computer-implemented manner based on the evaluation (6),
- wherein at least one optimized set value for the at least one action parameter (3) is independently learned by the apparatus (1) in a computer-implemented manner based on several evaluations (6) derived by the apparatus (1) from a respective process response (4), in each case in reaction to set values of the at least one action parameter (3) specified by the apparatus (1) .

2. Method according to Claim 1, wherein the preset target value (5) is a reference response (5) for the process response (4),
- in order in this manner to align the process response (4) with the preset target value (5).

3. Method according to Claim 1 or Claim 2, wherein the process (2) takes place in a sample (12) that comprises a culture medium (13) and the process response (4) is measured in the culture medium (13), in particular with exclusion of cells and/or microorganisms of the biological sample (12).

4. Method according to one of the preceding claims, wherein the process response (4) of the process (2) is measured on a medium (13) that surrounds elements (14) of the system (34) on which the process (2) is based, in particular wherein the system (34) is embedded in the medium (13) forming a culture medium (13).

5. Method according to one of the preceding claims, wherein at least one measurement parameter (8) of the process response (4) is measured in an immediate environment of the elements (14) of the biological system (34) on which the process (2) is based, but without direct measurement of the elements (14) of the biological system (34).

6. Method according to one of the preceding claims, wherein the consumption and/or the production of at least one material is acquired by the process (2), while the process (2) is influenced by setting the at least one action parameter (3).

7. Method according to one of the preceding claims, wherein the activity of the biological system (34), which is acquired by measuring the process response (4), is
- a thermogenesis, such as, for example, the production of heat by metabolic activity, and/or
- a chemo- or biogenesis, such as, for example, the production of chemical substances or biological organisms by metabolic activity, and/or
- a photogenesis, such as, for example, the production of light by metabolic activity, and/or
- an energy consumption and/or
- a material consumption or a material production.

8. Method according to one of the preceding claims, wherein the process (2) changes at least one environmental factor, for example
- a material composition and/or
- temperature and/or
- pH and/or
- permittivity and/or
- electrical conductivity and/or
- an optical transmission or reflection behaviour and/or
- an environmental factor of a culture medium (13) in which the biological system (34) is cultured,
and wherein the at least one environmental factor is measured as at least one measurement parameter (8) of the process response (4) in order to acquire a change in the at least one environmental factor in reaction to the adjustment of the at least one action parameter (3) .

9. Method according to one of the preceding claims, wherein at least one measurement parameter (8a) of the process response (4) is acquired by means of a spectrometric measurement,
- in particular of a culture medium (13) in which the biological system (34) is cultured.

10. Method according to Claim 9, wherein, for this purpose, only the culture medium (13), but not the elements (14) of the biological system (34) contained therein, are illuminated, or both the culture medium (13) and the elements (14) contained therein are illuminated.

11. Method according to one of the preceding claims, wherein the adjustment of the respective set value of the at least one action parameter (3) takes place by means of an artificial intelligence in a computer-implemented manner, in particular by means of a method of machine learning.

12. Method according to Claim 11,
- wherein the artificial intelligence uses data sets comprising
- a respective measured process response (4) and
- an accompanying set of action parameter values producing this process response (4)
in order to prepare and/or optimize a prediction model for the system response (4),
- preferably wherein the artificial intelligence, with the aid of the prediction model, generates virtual system responses in reaction to respective virtual sets of action parameter values.

13. Method according to Claim 12,
- wherein the artificial intelligence validates the virtual system responses, in particular after selection based on the preset target value (5), by means of real tests,
- preferably in that the artificial intelligence
- controls the apparatus (1) with the respective virtual set of action parameters,
- measures the real process response (4) arising therefrom, and
- compares it with the respective pre-calculated virtual system response.

14. Method according to one of Claims 11 to 13, wherein the at least one optimized set value for the at least one action parameter (3) is independently learned in a computer-implemented manner by the artificial intelligence based on the several evaluations (6).

15. Method according to one of the preceding claims, wherein, in addition to the process response (4), a process state is measured, in particular wherein a first measurement parameter (8) is measured by the process response (4) and a second measurement parameter (8) is measured by the process state.

16. Method according to one of the preceding claims, wherein at least two measurement parameters (8) are measured and wherein one of the two measurement parameters (8) is used in order to verify an evaluation carried out by means of the other measurement parameter (8),
- in particular wherein the evaluation is changed depending on a result of the verification, preferably by an, in particular the, artificial intelligence.

17. **Apparatus (1) for the autonomous control of a process (2),** for example a growth or a metabolism of a biological system (34), or for example wherein the process (2) is a chemical or a physical process, wherein the apparatus (1) comprises:
- a process chamber (15) for accommodating a biological, chemical or physical system, in which the process (2) takes place,
- a controller (16) configured to set at least one action parameter (3) in order to control the process (2) by means of the action parameter (3), and
- at least one measuring means (17), in particular
- a spectrometer (18) and/or
- a camera (19),
for measuring a process response (4) of the process (2), which takes place in reaction to an adjustment of the at least one action parameter (3), **characterized in that,** by measuring the process response (4),
- a consumption and/or a production of at least one material by the process can be, at least indirectly, acquired (2) and/or
- an activity of a biological system (34), which carries out the process, can be acquired, and
- the controller (16) is configured to independently learn at least one optimized set value for the at least one action parameter (3) in a computer-implemented manner based on several evaluations (6),
- wherein the apparatus (1) has derived the evaluations (6) from a respective process response (4), in each case in reaction to set values of the at least one action parameter (3) specified by the apparatus (1).

18. Apparatus (1) according to the preceding claim and configured to control a biological process (2), wherein at least one of the measuring means (17) is configured to acquire an overall measurement parameter (8) of the process response (4), which is able to be changed or is changed by all of the elements (14) of the biological system (34) on which the process (2) is based.

19. Apparatus according to Claim 17 or 18, wherein at least one of the measuring means (17),
- in particular a measuring means (17) configured to acquire the overall measurement parameter (8),
is arranged such that the measurement of the process response (4) takes place at least partially at a measuring point (20) that is kept free from the elements (14) of the biological system (34).

20. Apparatus (1) according to one of Claims 17 to 19, wherein the apparatus (1) comprises a microfluidic device (21) for supplying the system (34) with a medium, in particular a culture medium (13),
- preferably wherein the device (21) has a microfluidically active separating structure (22) with which the elements (14) of the biological system (34) are able to be kept distant from an, in particular the, measuring point (20), mentioned in Claim 19, at which the process response (4) is able to be acquired using the at least one measuring means (17).

## Revendications

1. **Procédé pour commander un appareil (1)** dans lequel un processus (2) se déroule,
- lequel processus (2) est commandé par le réglage d'au moins un paramètre d'action (3),
**caractérisé en ce**
- **qu'**une réponse de processus (4) du processus (2) à l'au moins un paramètre d'action (3) est mesurée,
- la mesure de la réponse de processus (4) ayant pour conséquence
- **qu'**une consommation et/ou une production d'au moins une matière par le processus (2) est détectée au moins indirectement et/ou
- **qu'**une activité d'un système biologique (34) qui exécute le processus est détectée,
- **que** la réponse de processus (4) mesurée est évaluée au moyen d'une évaluation (6) mise en oeuvre par ordinateur à l'aide d'un objectif (5) prédéfini pour la réponse de processus (4) mesurée, et
- **qu'**au moins une valeur de réglage de l'au moins un paramètre d'action (3) mis en oeuvre à l'aide de l'évaluation (6) est adaptée,
- au moins une valeur de réglage optimisée pour l'au moins un paramètre d'action (3) mis en oeuvre étant acquise de manière autonome par l'appareil (1) à l'aide de plusieurs évaluations (6) que l'appareil (1) a déduites d'une réponse de processus (4) concernée en réaction à des valeurs de réglage de l'au moins un paramètre d'action (3) prédéfinies par l'appareil (1).

2. Procédé selon la revendication 1, selon lequel l'objectif (5) est une réponse de référence (5) pour la réponse de processus (4),
- afin de comparer la réponse de processus (4) à l'objectif (5).

3. Procédé selon la revendication 1 ou la revendication 2, selon lequel le processus (2) se déroule dans un échantillon (12) qui comprend un milieu de culture (13) et la réponse de processus (4) est mesurée dans le milieu de culture (13), en particulier à l'exclusion de cellules et/ou de microorganismes de l'échantillon biologique (12).

4. Procédé selon une des revendications précédentes, selon lequel la réponse de processus (4) du processus (2) est mesurée sur un milieu (13) qui entoure des éléments (14) du système (34) à la base du processus (2), en particulier selon lequel le système (34) est immergé dans le milieu (13) constituant un milieu de culture (13).

5. Procédé selon une des revendications précédentes, selon lequel au moins un paramètre de mesure (8) de la réponse de processus (4) est mesuré dans un environnement direct des éléments (14) du système biologique (34) à la base du processus (2), mais sans mesure directe des éléments (14) du système biologique (34).

6. Procédé selon une des revendications précédentes, selon lequel la consommation et/ou la production d'au moins une matière par le processus (2) est détectée pendant qu'une influence est exercée sur le processus (2) par le biais du réglage de l'au moins un paramètre d'action (3).

7. Procédé selon une des revendications précédentes, selon lequel l'activité du système biologique (34) détectée par le biais de la mesure de la réponse de processus (4) est
- une thermogénèse, comme p. ex. la production de chaleur par une activité métabolique et/ou
- une chimiogénèse ou biogénèse, comme p. ex. la production de substances chimiques ou d'organismes biologiques par une activité métabolique et/ou
- une photogénèse, comme p. ex. la production de lumière par une activité métabolique et/ou
- une consommation d'énergie et/ou
- une consommation de matière.

8. Procédé selon une des revendications précédentes, selon lequel le processus (2) modifie au moins un facteur environnant, par exemple
- une composition matérielle et/ou
- une température et/ou
- une valeur pH et/ou
- une permittivité et/ou
- une conductivité électrique et/ou
- un comportement de transmission ou de réflexion optique et/ou
- un facteur environnant d'un milieu de culture (13) dans lequel le système biologique (34) est cultivé,
et selon lequel l'au moins un facteur environnant est mesuré comme au moins un paramètre de mesure (8) de la réponse de processus (4) afin de détecter une modification de l'au moins un facteur environnant en réaction à l'adaptation de l'au moins un paramètre d'action (3).

9. Procédé selon une des revendications précédentes, selon lequel au moins un paramètre de mesure (8a) de la réponse de processus (4) est détecté par le biais d'une mesure spectrométrique,
- en particulier d'un milieu de culture (13) dans lequel le système biologique (34) est cultivé.

10. Procédé selon une des revendications précédentes, selon lequel seul le milieu de culture (13) est exploré, mais pas les éléments (14) du système biologique (34) se trouvant à l'intérieur, ou aussi bien le milieu de culture (13) que les éléments (14) se trouvant à l'intérieur.

11. Procédé selon une des revendications précédentes, selon lequel l'adaptation de la valeur de réglage concernée de l'au moins un paramètre d'action (3) se fait au moyen d'une intelligence artificielle mise en oeuvre par ordinateur, en particulier au moyen d'une méthode d'apprentissage machine.

12. Procédé selon la revendication 11,
- selon lequel l'intelligence artificielle comprenant des jeux de données utilise
- une réponse de processus (4) mesurée concernée et
- un jeu correspondant de paramètres d'action générant cette réponse de processus (4)
pour mettre en place et/ou optimiser un modèle de pronostic pour la réponse du système (4),
- de préférence l'intelligence artificielle générant à l'aide du modèle de pronostic des réponses de système virtuelles en réaction à des jeux virtuels correspondants de paramètres d'action.

13. Procédé selon la revendication 12,
- selon lequel l'intelligence artificielle valide les réponses de système virtuelles, en particulier après sélection à l'aide de l'objectif (5), par des essais réels,
- de préférence l'intelligence artificielle
- commandant l'appareil (1) avec le jeu virtuel correspondant de paramètres d'action,
- mesurant la réponse de processus réelle (4) qui en découle et
- comparant celle-ci avec la réponse de système virtuelle correspondante calculée précédemment.

14. Procédé selon une des revendications 11 à 13, selon lequel l'au moins une valeur de réglage optimisée pour l'au moins un paramètre d'action (3) est acquise de manière autonome par l'intelligence artificielle, par mise en oeuvre par ordinateur, à l'aide des multiples évaluations (6).

15. Procédé selon une des revendications précédentes, selon lequel, outre la réponse de processus (4), un état de processus est mesuré, en particulier un premier paramètre de mesure (8) étant mesuré par la réponse de processus (4) et un deuxième paramètre de mesure (8) par l'état de processus.

16. Procédé selon une des revendications précédentes, selon lequel au moins deux paramètres de mesure (8) sont mesurés et selon lequel un des deux paramètres de mesure (8) est utilisé pour vérifier une évaluation réalisée au moyen de l'autre paramètre de mesure (8),
- en particulier l'évaluation étant modifiée en fonction d'un résultat de la vérification, de préférence par une intelligence artificielle, en particulier l'intelligence artificielle

17. **Appareil (1) de commande autonome d'un processus (2),** par exemple d'une croissance ou d'une activité métabolique d'un système biologique (34) ou, par exemple, le processus étant un processus chimique ou physique, lequel appareil (1) comprend
- une chambre de processus (15) pour recevoir un système biologique, chimique ou physique dans lequel le processus (2) se déroule,
- un contrôleur (16) conçu pour régler au moins un paramètre d'action (3) pour commander le processus (2) au moyen du paramètre d'action (3), et
- au moins un moyen de mesure (17), en particulier
- un spectromètre (18) et/ou
- une caméra (19),
pour mesurer une réponse de processus (4) du processus (2) qui a lieu en réaction à l'adaptation de l'au moins un paramètre d'action (3), **caractérisé en ce**
- **que**, par le biais de la mesure de la réponse de processus (4),
- une consommation et/ou une production d'au moins une matière par le processus (2) peut être détectée au moins indirectement et/ou
- une activité d'un système biologique (34) qui exécute le processus peut être détectée, et
- **que** le contrôleur (16) est conçu pour apprendre de façon autonome par mise en oeuvre par ordinateur, par le biais de plusieurs évaluations, au moins une valeur de réglage optimisée pour l'au moins un paramètre d'action (3), l'appareil (1) ayant déduit les évaluations (6) d'une réponse de processus (4) correspondante en réaction à des valeurs de réglage de l'au moins un paramètre d'action (3) prédéfinies par l'appareil (1).

18. Appareil (1) selon la revendication précédente et conçu pour commander un processus biologique (2), selon lequel au moins un des moyens de mesure (17) est conçu pour détecter un paramètre de mesure global (8) de la réponse de processus (4) qui peut être modifié ou est modifié par tous les éléments (14) du système biologique (34) qui est à la base du processus (2).

19. Appareil selon la revendication 17 ou 18 dans lequel au moins un des moyens de mesure (17),
- en particulier un moyen de mesure (17) conçu pour détecter le paramètre de mesure global (8),
est agencé de sorte que la mesure de la réponse de processus a lieu au moins partiellement sur un point de mesure (20) qui est dépourvu des éléments (14) du système biologique (34).

20. Appareil (1) selon une des revendications 17 à 19, lequel appareil (1) comprend un dispositif microfluidique (21) pour alimenter le système (34) avec un milieu, en particulier un milieu de culture (13),
- lequel dispositif (21) présente de préférence une structure de séparation (22) efficace microfluidiquement avec laquelle les éléments (14) du système biologique (34) peuvent être tenus à l'écart d'un point de mesure (20), mentionné en particulier dans la revendication 19, sur lequel la réponse de processus (4) peut être détectée avec l'au moins un moyen de mesure (17).
